# EUROPÄISCHE PATENTSCHRIFT

(11) **EP 1 636 185 B1**
(45) Veröffentlichungstag und Bekanntmachung des Hinweises auf die Patenterteilung: **11.01.2012**
(21) Anmeldenummer: 04737029.1
(22) Anmeldetag: 18.06.2004
(51) Int. Cl.: C07D 209/42

(54) **NEUE KRISTALLINE FORMEN VON PERINDOPRIL ERBUMINE**
NOVEL CRYSTALLINE FORMS OF PERINDOPRIL ERBUMINE
NOUVELLES FORMES CRISTALLINES DE PERINDOPRIL ERBUMINE

(30) Priorität: 24.06.2003 CH 110903
(43) Veröffentlichungstag der Anmeldung: 22.03.2006
(73) Patentinhaber: Les Laboratoires Servier, 92278 Neuilly sur Seine (FR)
(72) Erfinder: STRÄSSLER, Christoph, CH-8050 Zürich (CH); LELLEK, Vit, CH-8046 Zürich (CH); FÄSSLER, Roger, CH-8246 Langwiesen (CH)
(74) Vertreter: Braun, André jr.
(86) Internationale Anmeldenummer: PCT/CH2004/000374
(87) Internationale Veröffentlichungsnummer: WO 2004/113293

(56) Entgegenhaltungen:
- WO-A-01/83439
- WO-A-01/87835
- WO-A-01/87836
- WO-A-2004/046172
- US-A- 4 914 214

## Beschreibung

Die vorliegende Erfindung beschreibt zwei neue kristalline Formen von Perindopril erbumine.

Perindopril ((2S,3aS,7aS)-1-[2-(1-Ethoxycarbonyl-(S)-butylamino)-(S)-propionyl]-octahydroindol-2-carbonsäure) hat die folgende Formel (I):

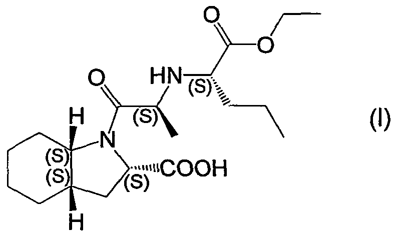

Perindopril erbumine ist das tert.-Butylaminsalz von Perindopril.

Perindopril wirkt als ACE-Inhibitor (ACE = Angiotensin Converting Enzyme) und wird zur Behandlung von kardiovaskulären Erkrankungen angewendet, im Speziellen zur Behandlung von Bluthochdruck und Herzinsuffizienz.

In EP 49658, US 4508729, EP 308341 und US 4914214 sowie in EP 1256590 und WO 01/58868 werden Synthesen für Perindopril beschrieben.

In WO 01/87835, WO 01/87836 und WO 01/83439 werden Kristallisationen von Perindopril erbumine aus Ethylacetat (WO 01/87835), aus Dichloromethan oder Ethylacetat (WO 01/87836) und aus Chloroform (WO 01/83439) und die dabei erhaltenen kristallinen Formen α (WO 01/87835), β (WO 01/87836) und γ (WO 01/83439) beschrieben.

Es wurde nun gefunden, dass durch Kristallisation von Perindopril erbumine einer beliebigen kristallinen Form aus tert.-Butylmethylether, welcher 0.9 - 2.5 % v/v Wasser enthält, bzw. durch Umformung der kristallinen Form α oder β von Perindopril erbumine in tert.-Butylmethylether, welcher 0.9 - 2.5 % v/v Wasser enthält, je nach den genauen

Bedingungen zwei weitere kristalline Formen δ und e erhalten werden können. Diese neuen kristalline Formen δ und ε werden durch folgende XRD-Daten charakterisiert (vgl. die nachstehenden Tabellen 1 und 2):

Die vorliegende Erfindung betrifft insbesondere ein Verfahren zur Herstellung von Perindopril erbumine der kristallinen δ-Form, dadurch gekennzeichnet, dass man Perindopril erbumine einer beliebigen kristallinen Form aus tert.Butylmethylether, welcher 0.9 - 1.4 % (v/v) Wasser oder 1.5 - 2.5 % (v/v) Wasser enthält, kristallisiert, wobei
- Perindopril erbumine einer beliebigen kristallinen Form aus tert.Butylmethylether, welcher 1.5 - 2.5 % (v/v) Wasser enthält, bei 30 - 45°C kristallisiert und den erhaltenen Niederschlag nach dem Entfernen von Wasser während mindestens 15 Stunden bei 30 - 45°C gerührt wird, oder
- dass Perindopril erbumine der kristallinen Form α oder β in tert.Butylmethylether, welcher 0.9 - 1.4 % (v/v) Wasser enthält, bei 33 - 38 °C unter Animpfen mit der kristallinen δ-Form gerührt wird, wobei die kristalline δ-Form von Perindopril erbumine, die folgenden in Tabelle 1 aufgeführten XRD-Daten (gemessen auf einem Pulverdiffraktometer mit CuKα Bestrahlung) aufweist:

**Tabelle 1:**

| Winkel 2 theta (°) | Gitterabstand d (Å) | Relative Intensität I/Iₘₐₓ (%) |
|---|---|---|
| 5.27 | 16.79 | 2 |
| 8.93 | 9.93 | 100 |
| 9.75 | 9.10 | 32 |
| 10.65 | 8.34 | 10 |
| 11.63 | 6.10 | 25 |
| 14.97 | 5.97 | 39 |
| 15.27 | 5.85 | 48 |
| 15.95 | 5.61 | 53 |
| 16.41 | 5.46 | 24 |
| 17.27 | 5.19 | 18 |
| 17.87 | 5.02 | 15 |
| 18.63 | 4.83 | 13 |
| 19.99 | 4.51 | 29 |
| 20.37 | 4.43 | 26 |
| 21.31 | 4.24 | 57 |
| 21.83 | 4.15 | 37 |
| 22.49 | 4.03 | 26 |
| 23.15 | 3.92 | 19 |
| 23.65 | 3.84 | 29 |
| 23.99 | 3.79 | 16 |
| 24.71 | 3.69 | 15 |
| 25.33 | 3.60 | 15 |
| 25.75 | 3.55 | 15 |
| 26.43 | 3.46 | 21 |
| 26.77 | 3.42 | 18 |
| 28.19 | 3.26 | 24 |

Anmerkung: Aufgrund von Textureffekten können die Intensitäten bekanntermassen variieren.

**Tabelle 2: XRD-Daten von Perindopril erbumine, kristalline Form ε (relative Intensitäten wurden dem durch CuK_{α}-Bestrahlung erhaltenen Pulverdiagramm entnommen).**

| Winkel 2 theta (°) | lattice spacing d (Å) | relative Intensität I/Iₘₐₓ (%) |
|---|---|---|
| 5.28 | 16.75 | 2 |
| 8.43 | 10.52 | 22 |
| 8.87 | 10.00 | 100 |
| 9.45 | 9.39 | 92 |
| 10.01 | 8.87 | 20 |
| 13.58 | 6.57 | 6 |
| 14.21 | 6.28 | 14 |
| 14.79 | 6.04 | 61 |
| 15.31 | 5.84 | 53 |
| 15.84 | 5.65 | 49 |
| 16.43 | 5.45 | 13 |
| 16.84 | 5.32 | 13 |
| 17.65 | 5.09 | 18 |
| 18.65 | 4.82 | 11 |
| 19.87 | 4.54 | 29 |
| 21.21 | 4.26 | 49 |
| 21.79 | 4.15 | 55 |
| 22.79 | 3.98 | 27 |
| 23.52 | 3.86 | 30 |
| 24.25 | 3.75 | 25 |
| 25.83 | 3.54 | 23 |
| 26.55 | 3.45 | 25 |
| 27.25 | 3.37 | 15 |
| 28.11 | 3.27 | 27 |

Anmerkung: Aufgrund von Textureffekten können die Intensitäten bekanntermassen variieren.

Die kristalline Form ε wird bei der Kristallisation von Perindopril erbumine einer beliebigen kristallinen Form aus tert.-Butylmethylether (MTBE), welcher 1.5 - 2.5% v/v Wasser enthält, bei 30 - 45°C, vorzugsweise 34 - 45°C, erhalten; diese Kristallisation erfolgt zweckmässigerweise unter Rühren. Wird dann das Wasser entfernt, zweckmässigerweise durch azeotrope Destillation, vorzugsweise bei 35 - 37°C, und wird dann für mindestens 15 h bei 30 - 45°C, vorzugsweise 35 - 37°C, weitergerührt, wandelt sich die kristalline Form ε in die kristalline Form δ um.

Zudem lassen sich die schon bekannten kristallinen Formen α und β von Perindopril erbumine durch Rühren in tert.-Butylmethylether (MTBE), welcher 0.9 - 1.4% v/v, vorzugsweise 1.0 - 1.1% v/v, Wasser, enthält, bei 33 - 38°C, vorzugsweise 35 - 37°C, und Animpfen mit der kristallinen Form δ in die kristalline Form δ umwandeln.

In tert.-Butylmethylether (MTBE) welcher 0.9 - 1.4% v/v, vorzugsweise 1.0 - 1.1% v/v, Wasser enthält, lassen sich bei 28 - 35°C, vorzugsweise 31 - 33°C, die schon bekannten Formen α und β durch Animpfen mit der kristallinen Form ε in die kristalline Form ε umwandeln.

Diese Umwandlungen lassen sich auch ohne Animpfen durchführen, allerdings kann im Grenzbereich nicht mit Sicherheit vorausgesagt werden, ob die Form δ, die Form ε oder Mischungen dieser Formen erhalten werden.

Die Umwandlung der kristallinen Formen α und β in die kristalline Form ε lässt sich auch bei 35 - 38°C in tert.-Butylmethylether (MTBE) welcher 1.5 - 2.0% v/v Wasser enthält, durchführen.

Die beiden kristallinen Formen δ und ε von Perindopril erbumine können als therapeutische Wirkstoffe verwendet und zusammen mit einem pharmazeutisch akzeptablen Trägermaterial zu einem Medikament verarbeitet werden. Dieses Medikament kann dann zur Behandlung von kardiovaskulären Krankheiten verwendet werden, im Speziellen zur Behandlung von Bluthochdruck und Herzinsuffizienz.

Pharmazeutisch akzeptable Trägermaterialien zur Herstellung von Medikamenten sind allgemein bekannt und jedem Fachmann geläufig.

Da verschiedene Formen eines pharmazeutischen Wirkstoffs, wie zum Beispiel neue kristalline Formen, in der Regel verschiedene Bioverfügbarkeiten, Löslichkeiten und Lösungsgeschwindigkeiten haben, können sie für die betreffenden Patienten von grossem Nutzen sein, da sie es vielleicht ermöglichen, die Dosis zu verkleinern oder die Dosisintervalle zu vergrössern und dadurch die Medikamentenkosten zu senken.

Die folgenden Beispiele sollten die Erfindung illustrieren.

Die XRD-Spektren wurden auf einem Philips ADP1700 Pulverdiffraktometer gemessen bei einer Cu-Bestrahlung von K_{α1} = 0.15406 nm und K_{α2} = 0.15444 nm und einer Spannung von 40 kV.

### Beispiel 1: Kristalline Form ε von Perindopril erbumine

5.00 g Perindopril erbumine wurden in 50 ml MTBE suspendiert und mit 0.95 ml Wasser versetzt. Die erhaltene Suspension wurde unter Rühren auf 48°C erwärmt, wobei sich eine klare Lösung bildete. Diese Lösung wurde unter Rühren auf 41°C abgekühlt. Bei dieser Temperatur wurde angeimpft, wonach die Kristallisation begann. Während 30 min wurde bei 40 - 41°C gerührt, und dann wurde während 1 h auf 34°C abgekühlt. Der entstandene Niederschlag wurde abfiltriert und mit 10 ml MTBE gewaschen. Nach dem Trocknen wurden 1.85 g Perindopril erbumine der kristallinen Form ε erhalten.

### Beispiel 2: Kristalline Form δ von Perindopril erbumine

11.09 g Perindopril erbumine wurden in 130 ml MTBE suspendiert und mit 2 ml Wasser versetzt. Die erhaltene Suspension wurde unter Rühren auf 51°C erwärmt, wobei sich eine klare Lösung bildete. Diese Lösung wurde während 120 min unter Rühren auf 35°C abgekühlt. Bei 44°C wurde angeimpft, wonach die Kristallisation begann. Bei 35 - 37°C wurden unter reduziertem Druck während 45 min 50 ml MTBE abdestilliert. Dabei wurde auch das Wasser azeotrop entfernt. Dann wurden nochmals 50 ml MTBE zugegeben und bei 35 - 37°C wurden nochmals 50 ml MTBE unter reduziertem Druck während 60 min abdestilliert. Bei 35 - 37°C wurde während 15 h weitergerührt und dann der erhaltene Niederschlag abfiltriert und mit 10 ml MTBE gewaschen. Nach dem Trocknen wurden 8.42 g Perindopril erbumine der Kristallinen Form δ erhalten.

### Beispiel 3: Umwandlung der kristallinen Form α in die kristalline Form δ

8.50 g Perindopril erbumine der kristallinen Form α wurden in 85 ml MTBE suspendiert und unter Rühren auf 35 - 37°C erwärmt. Dazu wurde 0.85 ml Wasser gegeben, gefolgt von 0.17 g Impfkristallen der kristallinen Form δ. Die erhaltene Suspension wurde 23 h bei 35 - 37°C gerührt und dann wurde der Niederschlag abfiltriert. Nach dem Trockenen wurden 7.00 g der kristallinen Form δ erhalten.

### Beispiel 4: Umwandlung der kristallinen Form α in die kristalline Form ε

21.66 g Perindopril erbumine der kristallinen Form α wurden in 216 ml MTBE suspendiert und unter Rühren auf 33 - 35°C erwärmt. Dazu wurde 2.16 ml Wasser gegeben und die erhaltene Suspension wurde 14 h bei 33 - 35°C gerührt. Nach Abfiltrieren und Trocknen des Niederschlages wurden 18.68 g der kristallinen Form ε erhalten.

### Beispiel 5: Umwandlung der kristallinen Form β in die kristalline Form δ

4.00 g Perindopril erbumine der kristallinen Form β wurden in 40 ml MTBE suspendiert und mit 0.36 ml Wasser versetzt. Die erhaltene Suspension wurde auf 35 - 37°C erwärmt und 20 h bei 35 - 37°C gerührt. Nach Abfiltrieren und Trocknen des Niederschlages wurde die kristalline Form δ erhalten.

### Beispiel 6: Umwandlung der kristallinen Form α in die kristalline Form ε

7.55 g Perindopril erbumine der kristallinen Form α wurden in 75 ml MTBE suspendiert und unter Rühren auf 35 - 37°C erwärmt. Dazu wurde 1.32 ml Wasser gegeben und die erhaltene Suspension wurde 20 h bei 35 - 37°C gerührt. Nach Abfiltrieren und Trocknen des Niederschlages wurden 2.31 g der der kristallinen Form ε erhalten.

## Patentansprüche

1. Verfahren zur Herstellung von Perindopril erbumine der kristallinen δ-Form, **dadurch gekennzeichnet, dass** man Perindopril erbumine einer beliebigen kristallinen Form aus tert.Butylmethylether, welcher 0.9 - 2.5 % (v/v) Wasser enthält, kristallisiert, wobei man
- Perindopril erbumine einer beliebigen kristallinen Form aus tert.Butylmethylether, welcher 1.5 - 2.5 % (v/v) Wasser enthält, bei 30 - 45°C kristallisiert und den erhaltenen Niederschlag nach dem Entfernen von Wasser während mindestens 15 Stunden bei 30 - 45°C rührt, oder
- dass Perindopril erbumine der kristallinen Form α oder β in tert.Butylmethylether, welcher 0.9 - 1.4 % (v/v) Wasser enthält, bei 33 - 38 °C unter Animpfen mit der kristallinen δ-Form gerührt wird, wobei die kristalline δ-Form von Perindopril erbumine, die folgenden XRD-Daten (gemessen auf einem Pulverdiffraktometer mit CuKα Bestrahlung) aufweist:
| Winkel 2 theta (°) | Gitterabstand d (Å) | Relative Intensität I/Iₘₐₓ (%) |
|---|---|---|
| 5.27 | 16.79 | 2 |
| 8.93 | 9.93 | 100 |
| 9.75 | 9.10 | 32 |
| 10.65 | 8.34 | 10 |
| 14.63 | 6.10 | 25 |
| 14.97 | 5.97 | 39 |
| 15.27 | 5.85 | 48 |
| 15.95 | 5.61 | 53 |
| 16.41 | 5.46 | 24 |
| 17.27 | 5.19 | 18 |
| 17.87 | 5.02 | 15 |
| 18.63 | 4.83 | 13 |
| 19.99 | 4.51 | 29 |
| 20.37 | 4.43 | 26 |
| 21.31 | 4.24 | 57 |
| 21.83 | 4.15 | 37 |
| 22.49 | 4.03 | 26 |
| 23.15 | 3.92 | 19 |
| 23.65 | 3.84 | 29 |
| 23.99 | 3.79 | 16 |
| 24.71 | 3.69 | 15 |
| 25.33 | 3.60 | 15 |
| 25.75 | 3.55 | 15 |
| 26.43 | 3.46 | 21 |
| 26.77 | 3.42 | 18 |
| 28.19 | 3.26 | 24 |

2. Verfahren zur Herstellung eines Arzneimittels enthaltend die kristalline 5-Form von Perindopril erbumine, beinhaltend, dass man Perindopril erbumine einer beliebigen kristallinen Form aus tert.Butylmethylether, welcher 0.9 - 2.5 % (v/v) Wasser enthält, kristallisiert, wobei man
- Perindopril erbumine einer beliebigen kristallinen Form aus tert.Butylmethylether, welcher 1.5 - 2.5 % (v/v) Wasser enthält, bei 30 - 45°C kristallisiert und den erhaltenen Niederschlag nach dem Entfernen von Wasser während mindestens 15 Stunden bei 30 - 45°C rührt, oder
- dass Perindopril erbumine der kristallinen Form α oder β in tert.Butylmethylether, welcher 0.9 - 1.4 % (v/v) Wasser enthält, bei 33 - 38 °C unter Animpfen mit der kristallinen δ-Form gerührt wird, wobei die kristalline δ-Form von Perindopril erbumine, die folgenden XRD-Daten (gemessen auf einem Pulverdiffraktometer mit CuKα Bestrahlung) aufweist:
| Winkel 2 theta (°) | Gitterabstand d (Å) | Relative Intensität I/Iₘₐₓ (%) |
|---|---|---|
| 5.27 | 16.79 | 2 |
| 8.93 | 9.93 | 100 |
| 9.75 | 9.10 | 32 |
| 10.65 | 8.34 | 10 |
| 14.63 | 6.10 | 25 |
| 14.97 | 5.97 | 39 |
| 15.27 | 5.85 | 48 |
| 15.95 | 5.61 | 53 |
| 16.41 | 5.46 | 24 |
| 17.27 | 5.19 | 18 |
| 17.87 | 5.02 | 15 |
| 18.63 | 4.83 | 13 |
| 19.99 | 4.51 | 29 |
| 20.37 | 4.43 | 26 |
| 21.31 | 4.24 | 57 |
| 21.83 | 4.15 | 37 |
| 22.49 | 4.03 | 26 |
| 23.15 | 3.92 | 19 |
| 23.65 | 3.84 | 29 |
| 23.99 | 3.79 | 16 |
| 24.71 | 3.69 | 15 |
| 25.33 | 3.60 | 15 |
| 25.75 | 3.55 | 15 |
| 26.43 | 3.46 | 21 |
| 26.77 | 3.42 | 18 |
| 28.19 | 3.26 | 24 |

3. Verfahren gemäss Anspruch 2, wobei das Arzneimittel zur Behandlung von kardiovaskulären Erkrankungen verwendet wird.

4. Verfahren gemäss Anspruch 3, wobei die kardiovaskulären Erkrankungen Bluthochdruck und Herzinsuffizienz sind.

## Claims

1. A method for preparing perindopril erbumine of the crystalline δ form, **characterized in that** perindopril erbumine of any given crystalline form is crystallized from tert-butyl methyl ether containing 0.9-2.5% (v/v) water, wherein
- Perindopril erbumine of any given crystalline form is crystallized at 30-45°C from tert-butyl methyl ether containing 1.5-2.5% (v/v) water, and the precipitate which is obtained, after removing water, is stirred for at least 15 hours at 30-45°C, or
- Perindopril erbumine of the α or β crystalline form is stirred at 33-38°C in tert-butyl methyl ether containing 0.9-1.4% (v/v) water, with inoculation with the crystalline δ form, wherein the crystalline δ form of perindopril erbumine exhibits the following XRD data (measured on a powder diffractometer using CuKα radiation):
| Angle 2-theta (°) | Lattice spacing d (Å) | Relative intensity I/Imax (%) |
|---|---|---|
| 5.37 | 16.19 | 2 |
| 8.93 | 9.93. | 100 |
| 9.75 | 9.10 | 32 |
| 10.65 | 8.34 | 10 |
| 14.63 | 6.10 | 25 |
| 14.97 | 5.97 | 39 |
| 15.27 | 5.85 | 48 |
| 15.95 | 5.61 | 53 |
| 16.41 | 5.46 | 24 |
| 17.27 | 5 19 | 18 |
| 17.87 | 5.02 | 15 |
| 18.63 | 4.83 | 13 |
| 19.99 | 4.51 | 29 |
| 20.37 | 4.43 | 26 |
| 21.31 | 4.24 | 57 |
| 21.83 | 4 15 | 37 |
| 22.49 | 4.03 | 26 |
| 23.15 | 3.92 | 19 |
| 23 65 | 3.84 | 29 |
| 13.99 | 3.79 | 16 |
| 24.71 | 3.69 | 15 |
| 25.33 | 3.60 | 15 |
| 25.75 | 3.55 | 15 |
| 26.43 | 3.46 | 21 |
| 26.77 | 3.42 | 18 |
| 28.19 | 3.26 | 24 |

2. A method for preparing a medicament containing the crystalline δ form of perindopril erbumine, wherein perindopril erbumine of any given crystalline form is crystallized from *tert-*butyl methyl ether containing 0.9-2.5% (v/v) water, wherein
- Perindopril erbumine of any given crystalline form is crystallized at 30-45°C from *tert-*butyl methyl ether containing 1.5-2.5% (v/v) water, and the precipitate which is obtained, after removing water, is stirred for at least 15 hours at 30-45°C, or
- Perindopril erbumine of the crystalline α or β form is stirred at 33-38 °C in tert-butyl methyl ether containing 0.9-1.4% (v/v) water, with inoculation with the crystalline δ form, wherein the crystalline δ form of perindopril erbumine exhibits the following XRD data (measured on a powder diffractometer using CuKα radiation):
| Angle 2-theta (°) | Lattice spacing d (Å) | Relative intensity I/Imax (%) |
|---|---|---|
| 5.27 | 16.79 | 2 |
| 8.93 | 9.93 | 100 |
| 9.75 | 9.10 | 32 |
| 10.65 | 8.34 | 10 |
| 14.63 | 6.10 | 25 |
| 14.97 | 5.97 | 39 |
| 15.27 | 5.85 | 48 |
| 15.95 | 5.61 | 53 |
| 16.41 | 5.46 | 24 |
| 17.27 | 5.19 | 18 |
| 17.87 | 5.02 | 15 |
| 18.63 | 4.83 | 13 |
| 19.99 | 4.51 | 29 |
| 20.37 | 4.43 | 26 |
| 21.31 | 4.24 | 57 |
| 21.83 | 4.15 | 37 |
| 22.49 | 4.03 | 26 |
| 23.15 | 3.92 | 19 |
| 23.65 | 3.84 | 29 |
| 23.99 | 3.79 | 16 |
| 24.71 | 3.69 | 15 |
| 25.33 | 3.60 | 15 |
| 25.75 | 3.55 | 15 |
| 26.43 | 3.46 | 21 |
| 26.77 | 3.42 | 18 |
| 28.19 | 3.26 | 24 |

3. A method according to Claim 2, wherein the medicament is used for treating cardiovascular diseases.

4. A method according to Claim 3, wherein the cardiovascular diseases are high blood pressure and cardiac insufficiency.

## Revendications

1. Procédé de fabrication de la Périndopril erbumine de la forme cristalline δ, **caractérisé par le fait que** l'on fait cristalliser de la Périndopril erbumine d'une forme cristalline quelconque dans le tert-butyl méthyl éther qui contient 0,9 - 2,5 % (v/v) d'eau, où
- l'on fait cristalliser à 30 - 45°C de la Périndopril erbumine d'une forme cristalline quelconque dans le tert-butyl méthyl éther qui contient 1,5 - 2,5 % (v/v) d'eau, et l'on agite le précipité obtenu après l'élimination de l'eau pendant au moins 15 heures à 30 - 45°C, ou
- l'on agite de la Périndopril erbumine de la forme cristalline α ou β dans le tert-butyl méthyl éther qui contient 0,9 - 1,4 % (v/v) d'eau, à 33 - 38°C sous inoculation avec la forme cristalline δ,
la forme cristalline δ de la Périndopril erbumine présentant les données de diffraction des rayons X suivantes (mesurées sur un diffractomètre de poudres avec un rayonnement CuKα) :
| Angle 2 thêta (°) | Pas du réseau d (Å) | Intensité relative I/Iₘₐₓ (%) |
|---|---|---|
| 5,27 | 16,79 | 2 |
| 8,93 | 9,93 | 100 |
| 9,75 | 9, 10 | 32 |
| 10, 65 | 8,34 | 10 |
| 14, 63 | 6,10 | 25 |
| 14, 97 | 5,97 | 39 |
| 15,27 | 5,85 | 48 |
| 15,95 | 5, 61 | 53 |
| 16,41 | 5,46 | 24 |
| 17,27 | 5,19 | 18 |
| 17, 87 | 5,02 | 15 |
| 18, 63 | 4,83 | 13 |
| 19,99 | 4,51 | 29 |
| 20,37 | 4,43 | 26 |
| 21,31 | 4,24 | 57 |
| 21,83 | 4,15 | 37 |
| 22,49 | 4,03 | 26 |
| 23,15 | 3,92 | 19 |
| 23, 65 | 3, 84 | 29 |
| 23,99 | 3,79 | 16 |
| 24,71 | 3, 69 | 15 |
| 25,33 | 3, 60 | 15 |
| 25,75 | 3,55 | 15 |
| 26,43 | 3,46 | 21 |
| 26,77 | 3,42 | 18 |
| 28,19 | 3,26 | 24 |

2. Procédé de fabrication d'un médicament contenant la forme cristalline δ de la Périndopril erbumine, suivant lequel on fait cristalliser de la Périndopril erbumine d'une forme cristalline quelconque dans le tert-butyl méthyl éther qui contient 0,9 - 2,5 % (v/v) d'eau, où
- l'on fait cristalliser à 30 - 45°C de la Périndopril erbumine d'une forme cristalline quelconque dans du tert-butyl méthyl éther, qui contient 1,5 - 2,5 % (v/v) d'eau, et l'on agite le précipité obtenu après l'élimination de l'eau pendant au moins 15 heures à 30 - 45°C, ou
- l'on agite de la Périndopril erbumine de la forme cristalline α ou β dans le tert-butyl méthyl éther qui contient 0,9 - 1,4 % (v/v) d'eau, à 33 - 38°C sous inoculation avec la forme cristalline δ,
la forme cristalline δ de la Périndopril erbumine présentant les données de diffraction des rayons X suivantes (mesurées sur un diffractomètre de poudres avec un rayonnement CuKα) :
| Angle 2 thêta (°) | Pas du réseau d (Å) | Intensité relative I/Iₘₐₓ (%) |
|---|---|---|
| 5,27 | 16,79 | 2 |
| 8,93 | 9,93 | 100 |
| 9,75 | 9,10 | 32 |
| 10,65 | 8,34 | 10 |
| 14,63 | 6,10 | 25 |
| 14,97 | 5,97 | 39 |
| 15,27 | 5,85 | 48 |
| 15,95 | 5,61 | 53 |
| 16,41 | 5,46 | 24 |
| 17,27 | 5,19 | 18 |
| 17,87 | 5,02 | 15 |
| 18,63 | 4,83 | 13 |
| 19,99 | 4,51 | 29 |
| 20,37 | 4,43 | 26 |
| 21,31 | 4,24 | 57 |
| 21,83 | 4,15 | 37 |
| 22,49 | 4,03 | 26 |
| 23,15 | 3,92 | 19 |
| 23,65 | 3,84 | 29 |
| 23,99 | 3,79 | 16 |
| 24,71 | 3,69 | 15 |
| 25,33 | 3,60 | 15 |
| 25,75 | 3,55 | 15 |
| 26,43 | 3,46 | 21 |
| 26,77 | 3,42 | 18 |
| 28,19 | 3,26 | 24 |

3. Procédé selon la revendication 2, suivant lequel le médicament est utilisé pour le traitement de maladies cardiovasculaires.

4. Procédé selon la revendication 3, suivant lequel les maladies cardiovasculaires sont l'hypertension et l'insuffisance cardiaque.
